**Europäisches Patentamt**

**European Patent Office**

**Office européen des brevets**

(19)

(11) Numéro de publication : **0 130 915**
**B1**

(12) **FASCICULE DE BREVET EUROPÉEN**

(45) Date de publication du fascicule du brevet :
26.04.89

(51) Int. Cl.⁴ : **A 61 F   5/14,** A 61 F   5/01

(21) Numéro de dépôt : **84401385.4**

(22) Date de dépôt : **29.06.84**

---

(54) **Module transverse anti-adductus pour la correction de l'adduction de l'avant-pied.**

---

(30) Priorité : **29.06.83 FR 8310800**

(43) Date de publication de la demande :
**09.01.85 Bulletin 85/02**

(45) Mention de la délivrance du brevet :
**26.04.89 Bulletin 89/17**

(84) Etats contractants désignés :
**AT BE CH DE FR GB IT LI LU NL SE**

(56) Documents cités :
**FR–A– 1 179 374**
**FR–A– 2 268 533**
**US–A– 2 395 936**
**US–A– 2 428 342**
**US–A– 2 958 324**
**US–A– 3 834 377**
**US–A– 4 314 412**

(73) Titulaire : **Etablissements DUBREUIL et LAROUDIE**
**Société à responsabilité limitée dite:**
**32 Boulevard Victor Hugo**
**F-87000 Limoges (FR)**

(72) Inventeur : **Gabilly, Jean-Pierre**
**53, rue de Texonnieras**
**F-87270 Couzeix (FR)**

(74) Mandataire : **Gillard, Marie-Louise et al**
**Cabinet Beau de Loménie 55, Rue d'Amsterdam**
**F-75008 Paris (FR)**

EP 0 130 915 B1

Jouve, 18, rue St-Denis, 75001 Paris, France

## Description

La présente invention concerne un module transverse anti-adductus pour la correction de l'avant-pied de l'enfant et notamment du nouveau-né, · ledit module pouvant être utilisé en permanence comprenant :

1) une semelle (1) comportant deux appuis latéraux internes fixes, l'un antérieur (3), l'autre postérieur· (2),

2) un contre-appui (5) externe médian, qui est amovible transversalement par rapport à la semelle et constitue une enveloppe de la partie médiane supérieure du pied, permettant le maintien du pied au fond du module par son appui transversal et par une pression oblique de haut en bas ;

3) des moyens d'immobilisation du contre-appui (5) par rapport à la semelle (1) dans la position d'appui.

Le module transverse anti-adductus selon l'invention va être décrit en détail en références aux dessins annexés sur lesquels :

- FIG. 1 est une vue en perspective d'un mode de réalisation du module selon l'invention ;

- FIG. 2 est une coupe du module représenté sur la figure 1 selon II-II ;

- FIG. 3 est un mode d'immobilisation de la position du contre-appui par rapport à la semelle ;

- FIG. 4 est une coupe en élévation d'un autre mode d'immobilisation du contre-appui par rapport à la semelle ;

- FIG. 5 est une coupe selon V-V du module représenté sur la figure 4 ;

- FIG. 6 est une vue de dessous du module représenté sur la figure 4 ;

- FIG. 7 est un autre mode de réalisation du module selon l'invention ;

- FIG. 8 est une coupe selon VIII-VIII du module représenté sur la figure 7.

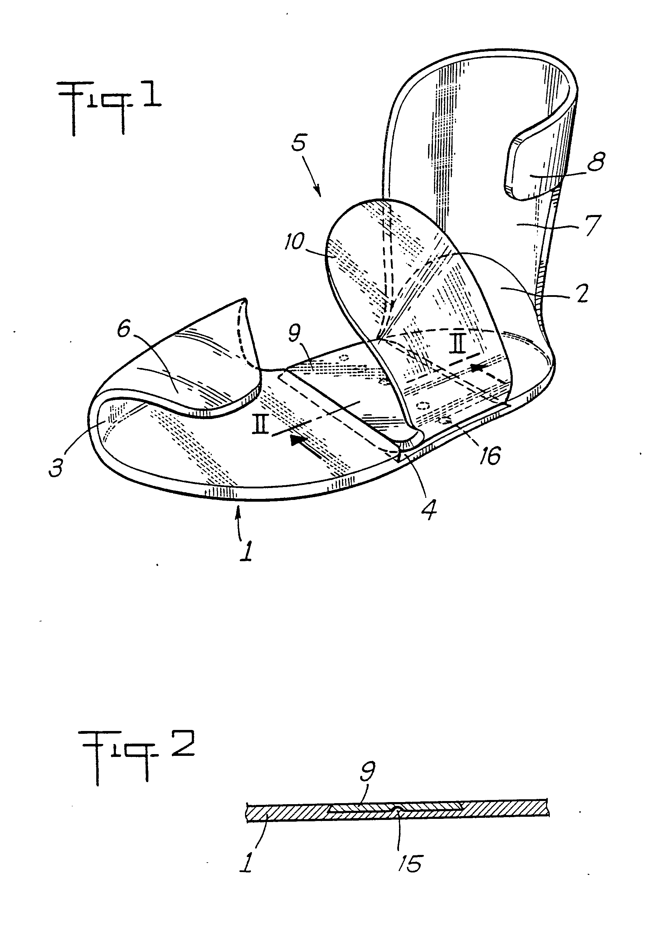

Le module anti-adductus représenté sur la figure 1 comprend a) une semelle 1 comportant un appui postérieur interne et latéral 2, un appui antérieur latéral et interne 3 et une rainure médiane transversale 4 ;

b) un contre-appui 5 médian externe et supérieur qui est amovible transversalement par rapport à la semelle 1. La semelle 1 et les appuis internes 2 et 3 sont agencés de manière à ce que le pied à corriger soit en abduction.

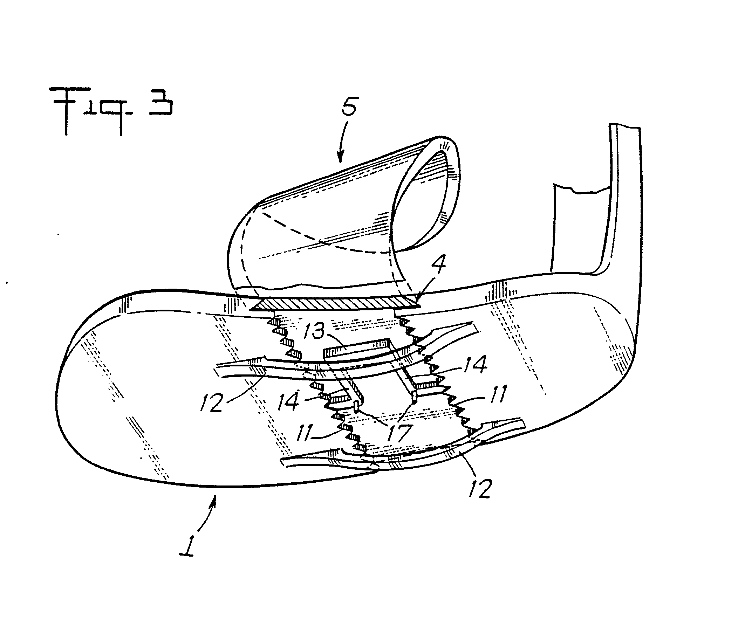

L'appui interne antérieur 3 se prolonge par une demi-enveloppe 6 de l'avant-pied et l'appui interne postérieur 2 est éventuellement prolongé par un tuteur interne 7, plus ou moins haut, muni d'un bracelet anti-équin 8. Le tuteur interne 7 peut être solidaire de l'appui 2 ou amovible par rapport à celui-ci, la fixation dudit tuteur 7 par rapport à l'appui 2 étant alors réalisée par des moyens classiques. Le contre-appui 5 représenté sur la figure 1 est un contre-appui rigide dont une partie 9 est plane et de dimensions appropriées pour coulisser dans la rainure transversale 4 de la semelle 1 et l'autre partie 10 est courbe de sorte que le contre-appui 5 constitue une enveloppe de la partie médiane externe du pied. Avantageusement, la rainure transversale 4 de la semelle 1 et la partie 9 du contre-appui 5 sont taillées en queue d'aronde, comme cela est représenté sur la coupe de la figure 2.

Le module selon l'invention permet l'inversion complète du pied grâce à l'abduction assurée par les appuis internes latéraux 2 et 3 et à la correction transversale exercée par le contre-appui 5.

Etant donné que le contre-appui 5 est amovible, le module de correction selon l'invention peut convenir pour toutes les longueurs et les largeurs de pied. De plus, pour un pied donné, la correction progressive de celui-ci peut être assurée avec le même module grâce au contre-appui amovible et réglable, contrairement aux systèmes orthopédiques connus, qui nécessitent le façonnage successif d'attelles orthopédiques.

Le pied atteint d'adduction est positionné sur la semelle 1 et fermement immobilisé par le contre-appui 5 en déplaçant celui-ci transversalement dans la rainure 4 de la semelle jusqu'à ce que la position adéquate de correction soit atteinte, le contre-appui 5 devant alors rester immobile par rapport à la semelle 1. Cette immobilisation ou position d'appui peut être assurée par tout système d'immobilisation du contre-appui 5 par rapport à la semelle 1. Un exemple d'un tel système peut être constitué par un ergot 15 demi-sphérique solidaire de la semelle 1 et situé par exemple dans la partie centrale de la rainure et d'une série de cavités demi-sphériques 16 de dimensions correspondantes, sur la surface inférieure de la partie 9 du contre-appui 5.

Les cavités 16 sont alignées et positionnées de manière à ce que, lorsque le contre-appui 5 est engagé dans la rainure 4, l'ergot 15 soit en regard de la ligne constitué par lesdites cavités 16. Ainsi, le contre-appui 5 déplacé dans la rainure 4 par pression transversale, reste immobile par rapport a la semelle, donc en position d'appui, dès que l'ergot de la rainure 4 est dans une cavité de la face inférieure du contre-appui 5.

Un autre exemple de système d'immobilisation qui convient aux fins de l'invention est constitué par un système de crémaillère avec friction et cliquet, éventuellement muni d'un système de ressort à lame ou en jonc procurant une intensité variable de la pression du contre-appui 5 par rapport à la semelle 1.

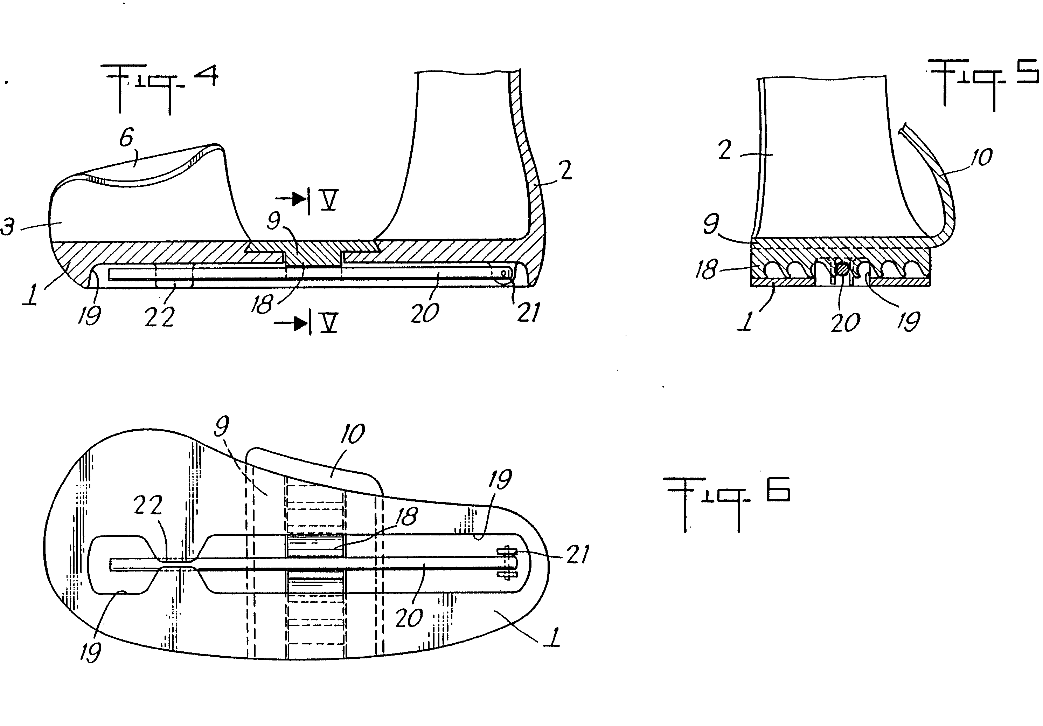

Un exemple d'immobilisation par système de crémaillère est représenté sur la figure 3 qui est une vue de dessous en perspective d'un module selon l'invention. Dans ce mode de réalisation, la rainure 4 est évidée sur toute sa longueur et munie de crans 11 constituant la .crémaillère ; étant donné l'évidement de la rainure 4, les parties avant et arrière de la semelle sont maintenues solidaires grâce aux ponts 12. Le contre-appui 5 comporte sur la face inférieure de sa partie 9 un tenon 13 muni de deux lamelles souples 14 qui viennent en appui dans les crans 11 de la crémaillère ; ces lamelles sont munies d'ergot de préhension 17 permettant de désolidariser le système de cliquet.

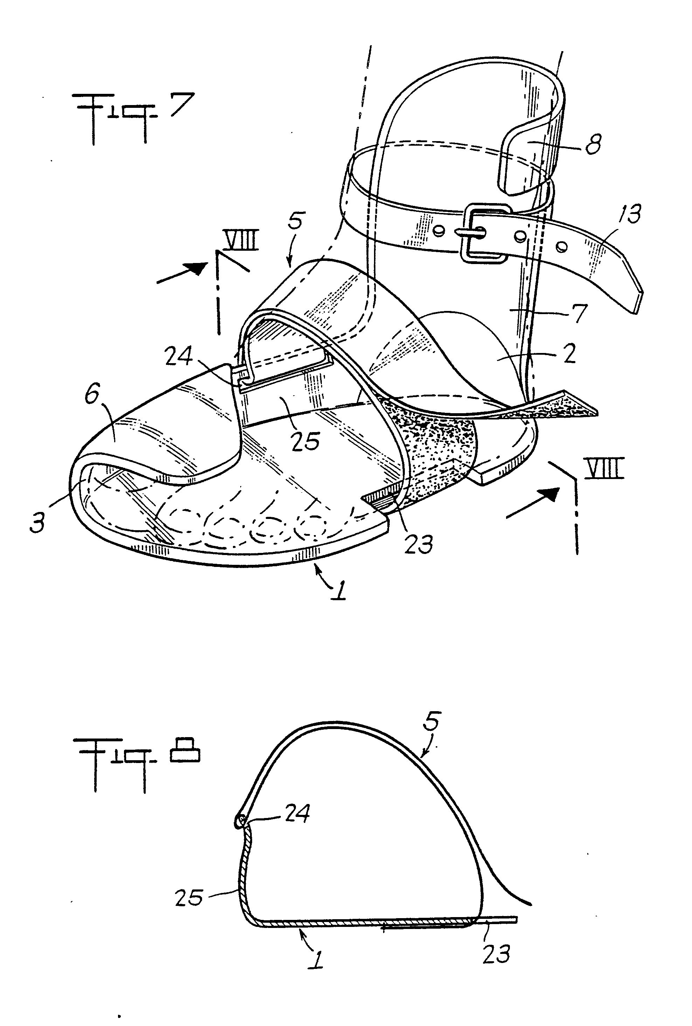

Une autre variante de réalisation du système d'immobilisation est représenté sur les figures 4 à 6, dans lequel le contre-appui 5 est muni sur la face inférieure de sa partie 9 d'un tenon strié 18 ; la face inférieure de la semelle 1 est partiellement évidée, par exemple comme représenté en 19 sur la figure 6 et est munie d'un ressort 20 articulé par des moyens appropriés en 21 et pouvant être fixé sous l'avant de la semelle dans une gorge 21. Le ressort 20 coopère avec les stries du tenon 18 pour maintenir la position d'appui désirée du contre-appui 5.

Comme indiqué précédemment, le contre-appui 5 du module selon l'invention peut être souple, constitué par exemple d'une bande. Un exemple de ce mode de réalisation selon l'invention est représenté sur la figure 7 sur laquelle 1 désigne la semelle du module, 3 l'appui antérieur interne latéral, 2 l'appui postérieur interne latéral et 6 la demi-enveloppe de l'avant-pied. Les bords antérieur 3 et postérieur 2 qui assurent les appuis internes latéraux antérieur et postérieur sont reliés par un bord médian interne 25 de sorte que, selon ce mode de réalisation, la semelle possède un bord interne sur toute sa longueur.

L'appui interne antérieur 2 comporte un tuteur interne 7 avec bracelet postérieur de la jambe anti-équin 8 muni éventuellement d'un bracelet de maintien. La partie médiane externe de la semelle comporte une échancrure rectangulaire 23 dont la longueur est sensiblement supérieure à celle du contre-appui 5. La bande constituant le contre-appui 5 est fixé sous la semelle 1 par des moyens appropriés, par exemple par collage. La face externe du bord médian 25 est munie de moyens pour faire coulisser le contre-appui souple.

Ces moyens peuvent être constitués par une fenêtre 24 ménagée dans le bord médian 25. Lors de l'utilisation, le pied à corriger est positionné sur la semelle et est mis dans la position de correction adéquate à l'aide de la bande constituant le contre-appui 5, que l'on passe sur la partie médiane supérieure du pied, qu'on enfile ensuite, dans la fenêtre 24 et qu'on ramène sur la partie médiane du pied, en la tirant suffisamment pour mettre le pied dans la position de correction désirée, laquelle position est maintenue en fixant l'une par rapport à l'autre les deux parties de la bande qui passe sur la partie médiane supérieure du pied ; cette fixation peut être assurée par exemple par des moyens d'auto-accrochage par contact de type « VELCRO » dont peut être munie la bande, partiellement ou sur pratiquement toute sa longueur.

Ce mode de réalisation du module selon l'invention convient particulièrement bien aux nouveau-nés.

La semelle du module selon l'invention, y compris les appuis latéraux internes sont constitués par exemple en cuir, en matériaux de synthèse, tels que les matières plastiques. Avantageusement, on utilise une matière plastique thermoformable, de préférence une matière thermoformable à basse température.

Dans le cas d'un contre-appui rigide, le contre-appui peut être constitué en le même matériau que la semelle. Le module selon l'invention peut également être réalisé en deux matériaux :
- la semelle 1 et la partie 9 du contre-appui 5, ainsi qu'éventuellement les tenons 14 et 18 et les lamelles 14 peuvent être en un matériau thermoformable à haute température.
- La partie 10 du contre-appui 5, les appuis antérieur et postérieur internes et le tuteur muni éventuellement du bracelet anti-équin peuvent être en un matériau thermoformable à basse température.

Cette variante permet la livraison d'un appareil préformé qui, trempé dans de l'eau chaude à 60 °C rend les pièces thermoformables à basse température maléables et donc adaptables aux variantes dimensionnelles directement sur le pied de l'enfant.

Lorsque le contre-appui est souple, il est avantageusement constitué d'une bande de tissu munie de moyens d'auto-accrochage. Le mode de réalisation particulièrement préféré du module de l'invention est celui représenté sur la figure 1, chaque partie (semelle et contre-appui) en matière plastique thermoformable est obtenue par moulage, injection ou tout autre procédé classique de réalisation d'objets en matières plastiques.

Avec le module selon l'invention, la correction est bien réalisée sur la diaphyse antérieure du premier métatarsien avec possibilité de laisser libre (ou moins correcteur) la première phalange distale du premier doigt. Il n'y a aucun pincement possible de la peau du pied à corriger.

La mise en place du pied est simple et rapide dans le module selon l'invention du fait que le contre-appui 5 est amovible. Le contre-appui 5 permet le maintien du pied au fond du module par son appui transversal et par une pression oblique de haut en bas, permettant ainsi le maintien du pied dans le module sans aucun autre système de fixation désagréable par la manipulation et inesthétique. D'autre part, la correction assurée par le module de l'invention est aussi bien contrôlable visuellement que radiologiquement.

Le module selon l'invention est adaptable sur une attelle de Denis BROWN. Grâce au thermoformage du tuteur et du bracelet, il est possible de rendre le module anti-équin et en pronation.

La correction progressive assurée par le contre-appui 5 est fixe ou variable dans la position d'appui ou de pression. Elle est variable si le module comporte des coussinets pneumatiques ou un système de ressort, comme défini sur les figures 4 à 6. Avantageusement, le module selon l'invention peut être recouvert d'une mince épaisseur de mousse non traumatisante.

**Revendications**

1. Module transverse anti-adductus pour la correction de l'avant-pied de l'enfant et notamment du nouveau-né, ledit module pouvant être utilisé en permanence comprenant :

1) une semelle (1) comportant deux appuis latéraux internes fixes, l'un antérieur (3), l'autre postérieur (2),

2) un contre-appui (5) externe médian, qui est amovible transversalement par rapport à la semelle et constitue une enveloppe de la partie médiane supérieure du pied, permettant le maintien du pied au fond du module par son appui transversal et par une pression oblique de haut en bas ;

3) des moyens d'immobilisation du contre-appui (5) par rapport à la semelle (1) dans la position d'appui.

2. Module selon la revendication 1, caractérisé en ce que la semelle (1) possède une rainure médiane transversale (4), en ce que le contre-appui (5) est rigide et constitué d'une partie plane (8) de dimensions appropriées pour coulisser dans la rainure (4) et d'une partie courbe (10) constituant l'enveloppe de la partie médiane externe du pied.

3. Module selon l'une des revendications 1 ou 2, caractérisé en ce que l'appui latéral interne et antérieur se prolonge par une demi-enveloppe (6) de l'avant-pied.

4. Module selon l'une des revendications 1 à 3, caractérisé en ce que l'appui latéral interne et postérieur (2) est muni d'un tuteur interne (7) et d'un bracelet anti-équin (8) éventuellement amovibles.

5. Module selon l'une des revendications 1 à 4, caractérisé en ce que la semelle et le contre-appui sont en un matériau plastique thermoformable.

6. Module selon l'une quelconque des revendications 1 à 5, caractérisé en ce que les moyens d'immobilisation du contre-appui (5) par rapport à la semelle (1) dans la position d'appui sont constitués d'une part d'un ergot (15) demi-sphérique situé dans la rainure (4) de la semelle (1) et d'une série de cavités (16) demi-sphériques sur la face inférieure de la partie (9) du contre-appui (5).

7. Module selon la revendication 1, caractérisée en ce que les moyens d'immobilisation du contre-appui (5) par rapport à la semelle (1) sont constitués d'un système de crémaillère.

8. Module selon l'une quelconque des revendications 1 à 7, caractérisé en ce que le système d'immobilisation est constitué d'un tenon strié (18) solidaire du contre-appui (5) et d'un ressort (20) articulé en (21) et pouvant être fixé dans la gorge (22), la face inférieure de la semelle étant partiellement évidée selon (19).

9. Module selon la revendication 1, caractérisé en ce que le contre-appui (5) est souple et constitué d'une bande munie de moyens d'auto-accrochage, en ce que la semelle comporte une échancrure rectangulaire (23) sur la partie médiane externe, en ce que les appuis latéraux internes (2) et (3) sont reliés par un bord médian (25), en ce que la face externe dudit bord médian (25) est munie d'une fenêtre (24).

## Claims

1. Transverse anti-adductus module for the correction of the fore-part of a child's foot, and in particular of a newborn child, which module can be used permanently, comprising :

1) a sole (1) comprising two inner lateral fixed supports, one anterior (3), the other posterior (2),

2) an outer median counter-support (5), which is movable transversally relative to the sole and constitutes an envelope of the upper median part of the foot, for holding the foot at the bottom of the module by its transversally applied pressure and by an oblique pressure applied downwardly from the top ;

3) means of immobilization of the counter-support (5) relative to the sole (1) in the support position.

2. Module according to claim 1, characterized in that the sole (1) has a transversal median groove (4), in that the countersupport (5) is rigid and constituted of a flat part (9) of appropriate dimensions to slide in the groove (4) and a curved part (10) constituting the envelope of the upper median part of the foot.

3. Module according to one of claims 1 or 2, characterized in that the anterior inner lateral support is extended by a semienvelope (6) for the fore-part of the foot.

4. Module according to one of claims 1 to 3, characterized in that the posterior inner lateral support (2) is equipped with an inner brace (7) and an optionally removable anti-equinus extension (8).

5. Module according to one of claims 1 to 4, characterized in that the sole and the counter-support are in a thermoshapable plastic material.

6. Module according to any one of claims 1 to 5, characterized in that the means of immobilization of the counter-support (5) relative to the sole (1) in the pressure position are constituted, on the one hand, of a semi-spherical lug (15) situated within the groove (4) of the sole (1) and of a series of semi-spherical cavities (18) on the lower face of the part (9) of the counter-support (5).

7. Module according to claim 1, characterized in that the means of immobilization of the counter-support (5) relative to the sole (1) are constituted of a rack system.

8. Module according to any one of claims 1 to 7, characterized in that the immobilization system is constituted of a channelled tenon (18) fast with the counter-support (5) and of a spring (20) articulated at (21) and able to be fixed in the groove (22), the lower face of the sole being partially hollowed out according to (19).

9. Module according to claim 1, characterized in that the counter-support (5) is flexible and constituted of a band provided with self-fastening means, in that the sole includes a rectangular indentation (23) along the outer median part, in that the inner lateral supports (2) and (3) are linked by a median edge (25), in that the outer face of the median edge (25) is provided with a window (24).

## Patentansprüche

1. Antiadduktions-Transversalregulator zur Korrektur des Vorfußes eines Kindes, insbesondere eines Neugeborenen, welche Regulator ständig verwendbar ist, umfassend :

1) eine Sohle (1) mit zwei feststehenden inneren Seitenstützen, u. zw. einer vorderen (3) und einer hinteren (2),

2) eine äußere mittlere Gegenstütze (5), die in bezug auf die Sohle in Querrichtung beweglich ist und eine Hülle für den oberen Mittelteil des Fußes bildet, die den Halt des Fußes am Boden des Regulators über seine Querstütze und durch Schrägdruck von oben nach unten gestattet ;

3) Mittel zum Immobilisieren der Gegenstütze (5) in bezug auf die Sohle (1) in der Stützposition.

2. Regulator nach Anspruch 1, dadurch gekennzeichnet, daß die Sohle (1) eine mittlere Querrille (4) besitzt, daß die Gegenstütze (5) starr ist und durch einen flachen Teil (9) von zum Gleiten in der Rille (4) geeigneten Dimensionen und einen gekrümmten Teil (10), der die Hülle für den äußeren Mittelteil des Fußes darstellt, gebildet ist.

3. Regulator nach einem der Ansprüche 1 oder 2, dadurch gekennzeichnet, daß die innere und vordere Seitenstütze durch eine Halbhülle (6) für den Vorfuß verlängert ist.

4. Regulator nach einem der Ansprüche 1 bis 3, dadurch gekennzeichnet, daß die innere und hintere Seitenstütze (2) mit einer Innenabstützung (7) und einem Ring (8) gegen Klumpfuß, die gegebenenfalls abnehmbar sind, versehen ist.

5. Regulator nach einem der Ansprüche 1 bis 4, dadurch gekennzeichnet, daß die Sohle und die Gegenstütze aus wärmeverformbarem Kunststoff sind.

6. Regulator nach einem der Ansprüche 1 bis 5, dadurch gekennzeichnet, daß die Mittel zum Immobilisieren der Gegenstütze (5) in bezug auf die Sohle (1) in der Stützposition einerseits durch eine halbkugelförmige, in der Rille (4) der Sohle (1) befindliche Nocke (15) und aus einer Reihe von halbkugelförmigen Vertiefungen (16) auf der Innenseite des Teils (9) der Gegenstütze (5) gebildet sind.

7. Regulator nach Anspruch 1, dadurch gekennzeichnet, daß die Mittel zum Immobilisieren der Gegenstütze (5) in bezug auf die Sohle (1) aus einem Zahnstangensystem bestehen.

8. Regulator nach einem der Ansprüche 1 bis 7, dadurch gekennzeichnet, daß das Immobilisationssystem durch einen mit der Gegenstütze (5) zusammenhängenden gerillten Fuß (18) und eine in (21) angelenkte und in der Nut (22) befestigbare Feder (20) gebildet ist, wobei die Unterseite der Sohle bei (19) teilweise ausgespart ist.

9. Regulator nach Anspruch 1, dadurch gekennzeichnet, daß die Gegenstütze (5) nachgiebig ist und aus einem Band besteht, das mit automatischen Verhakungsmitteln versehen ist, daß die Sohle eine rechteckige Ausnehmung (23) am äußeren Mittelteil aufweist, daß die inneren Seitenstützen (2) und (3) durch einen Mittelbord (25) miteinander verbunden sind, und daß die Außenfront des Mittelbordes (25) mit einer Öffnung (24) versehen ist.

Fig. 1

Fig. 2

Fig. 3

Fig. 4

Fig. 5

Fig. 6

EP 0 130 915 B1

Fig. 7

Fig. 8